# EUROPEAN PATENT SPECIFICATION

(11) **EP 2 512 217 B1**
(45) Date of publication and mention of the grant of the patent: **03.08.2022**
(21) Application number: 10793168.5
(22) Date of filing: 16.12.2010
(51) Int. Cl.: A01H 1/08, A01H 5/12

(54) **Tetraploid corn salad**
Tetraploider Feldsalat
Mâche tétraploïde

(30) Priority: 17.12.2009 EP 09015637
(43) Date of publication of application: 24.10.2012
(73) Proprietor: Nunhems B.V., 6083 AB Nunhem (NL)
(72) Inventor: HEUVELMANS, Paul, NL-5981 NM Panningen (NL)
(74) Representative: BASF IP Association
(86) International application number: PCT/EP2010/007661
(87) International publication number: WO 2011/072848

(56) References cited:
- WO-A1-99/03329
- US-A1- 2006 143 741
- US-A1- 2009 176 227
- US-B1- 6 320 104
- MUMINOVIC J ET AL: "Genetic diversity in cornsalad (Valerianella locusta) and related species as determined by AFLP markers", PLANT BREEDING, vol. 123, no. 5, October 2004 (2004-10), pages 460-466, XP002580359, ISSN: 0179-9541 cited in the application
- Hirokazu Tsukaya: "Controlling Size in Multicellular Organs: Focus on the Leaf", PLOS BIOLOGY, vol. 6, no. 7, 15 July 2008 (2008-07-15), page e174, XP055348349, US ISSN: 1544-9173, DOI: 10.1371/journal.pbio.0060174
- OSBORN T C ET AL: "Understanding mechanisms of novel gene expression in polyploids", TRENDS IN GENETICS, ELSEVIER SCIENCE PUBLISHERS B.V. AMSTERDAM, NL, vol. 19, no. 3, 1 March 2003 (2003-03-01), pages 141-147, XP004411460, ISSN: 0168-9525, DOI: 10.1016/S0168-9525(03)00015-5
- Mei Guo ET AL: "Dosage Effects on Gene Expression in a Maize Ploidy Series", Genetics, vol. 142, 1 April 1996 (1996-04-01), pages 1349-1355, XP055741071,

## Description

### FIELD OF THE INVENTION

The present invention relates to the field of plant breeding and plant improvement. Provided are tetraploid corn salad plants *(Valerianella locusta (L.) Betcke)* having improved characteristics, as well as methods for making tetraploid corn salad plants from diploid plants. Also provided are seeds from which tetraploid plants can be grown, as well as harvested, processed and/or packaged tetraploid plant parts (e.g. leaves), plantlets or whole plants.

### BACKGROUND OF THE INVENTION

Corn salad (or lamb's lettuce), *Valerianella locusta (L.) Betcke* (synonym formerly *Valerianella olitoria (L.) Poll.; fam. Valerianaceae)* is a leafy vegetable crop cultivated in several European countries, such as the Netherlands, Italy, Germany and France, as well as in parts of North America and Australia. Wild *V. locusta* is native to Europe, temperate western Asia and parts of North-Africa. Of almost 80 species classified in the genus *Valerianella,* only *V. locusta* is cultivated for human consumption. The leaves or whole plants or whole plantlets are used in fresh salads, often mixed with lettuce leaves *(Lactuca sativa)* and/or other fresh vegetables (e.g. rocket leaves - *Eruca sativa,* water cress, garden cress, *Raphanus* cress as in EP1290938B1, etc.). Production takes place in both glasshouse cultivation and field cultivation, optionally under covers (e.g. plastic tunnels or shade nettings). Also soilless culture systems and hydroponic culture systems have been described (see e.g. Fontana and Nicola, 2009, J of Food, Agriculture and Env. Vol 7: 405-410 or Benoit, F. and Ceustermans, N. 1989. Acta Hort. (ISHS) 242:297-304).

Production is possible all year round, with numerous varieties which are adapted to specific climatic conditions e.g. summer or winter.

Plants produce rosettes, presenting up to 8 pairs of opposite leaves, each pair being at a right-angle to the previous pair. Generally long-leaved and short-leaved ("rosette" type) varieties are distinguished. Breeding objectives in corn salad aim predominantly at the production of lines or varieties of the rosette type, with round, dark green leaves due to consumer preference. Corn salad is also known as lamb's lettuce. It is an autogamous (self-fertilizing), diploid crop (chromosome number is not clear; numbers of 2n = 14, 16 and 18 have been reported, with 2n = 16 being the most common report, see Löve and Kjellqvist 1974, Lagascalia 4:153-211), with a narrow genetic base in the currently available breeding germplasm (Muminovic et al. 2004, Plant Breeding 123: 460-466). The specification herein below will refer to 2n = 16, but it is understood that the embodiments equally apply in case 2n should turn out to be a different number, e.g. 14 or 18.

Harvesting of Corn salad leaves or whole plants can begin from about 25 to 110 days after sowing, depending on the variety, but mainly on growth conditions (see e.g. Peron and Rees, Acta Hort. 467, ISHS 1998, table 3 on page 264). Also, the post-harvesting processing and packaging determines the harvest time. For sealed (plastic) bags, for example, harvest may be when plants have about 3-4 pairs of leaves, while for sale in (sealed, plastic) punnets or sealed packets about 4-6 pairs of leaves, and for sale in open trays about 7-8 pairs of leaves, may be present. Harvest can be manual and/or mechanized, followed by manual and/or mechanical cleaning (using a series of washers or rinsers) and packaging (Peron and Rees, Acta Hort. 467, ISHS 1998, pp259-268; Geyer and Herppich, Gemuese, 1999 Vol. 35, 657-660, Feldsalat schonend, qualitätsgerecht und hygienisch aufbereiten).

Leafy vegetables such as Corn salad must be fresh and turgid to appeal to consumers, with no yellowing, damage or decay/rot (which often develops as a result of tissue damage). Minimal damaging during harvest and post-harvest processing and long shelf-life are, therefore, important for marketability. Equally, mouthfeel (crispiness or crunchiness) is an important quality criterion.

WO99/03329 discloses diploid multileaf lettuce and lamb's lettuce, whereby the plants have substantially higher numbers of leaves, but the size of the lambs lettuce plant is comparable to the normal lambs lettuce plants (see Example 3).

US2009/176227 relates to a method for production of polyploidy plants of orchids using cuttings and tissue culture.

US2006/143741 relates to a tetraploid Perennial Ryegrass variety, called T3.

The present inventors have surprisingly found that tetraploidisation of Corn salad varieties or breeding lines can be used to make Corn salad plants which have significantly thicker and/or firmer leaves than the diploid plant from which they are derived, without significantly enlarging plant size and/or leaf length, as set out in the appended claims. On the contrary: autotetraploid Corn salad plants and leaves are compacter than the diploid plants and/or diploid leaves. This finding was surprising, as tetraploidization is known to increase organ size. For example, Sugiyama (Annals of Botany 2005, 96: 931-938) studied the effect of tetraploidization of two species of *Lolium,* concluding that tetraploidy had a predominant effect on increasing leaf size (both leaf length and leaf width) compared to the diploids, which was due to an increase in cell elongation rate in the autotetraploids (while cell division was not affected). Sugimoto-Shirasu and Roberts review the relationship of endoreduplication at the cellular level and how ploidy influences cell-size. In animals polyploidy leads to larger cells, without affecting organ size (fewer cells are present in each organ), while plants do not seem to obey this rule and the authors mention that "tetraploid plants are invariably larger than their diploid relatives" (Current Opinion in Plant Biology 2003, 6: 544-553, see page 550, LH Column, first paragraph).

In addition it was surprising that tetraploidization was not found to be associated with common problems, such as fertility disturbances, disturbances during meiosis, mixoploidy or aneuploidy. Fertility and seed production of tetraploid Corn salad was normal.

It is, therefore, an objective to provide Corn salad plants and plant parts (and seeds from which such plants can be grown) which are compact and have thick leaves, as set out in the appended claims. It is a further objective of the invention to provide fresh Corn salad plants and/or leaves which have a crisp mouthfeel. Also, the use of autotetraploidization to generate Corn salad plants having thicker and/or compacter and/or crunchier leaves (compared to the leaves of the diploid plants from which they were derived) is provided herein.

### GENERAL DEFINITION

"Corn salad" refers herein to plants, seeds and/or plant parts (e.g. harvested leaves, plantlets, tissues or organs, cells, etc.) of the species *Valerianella locusta.*

"Crop plants" or "cultivated plants" or "non-naturally occurring plants" are cultivated breeding lines or varieties, which differ from wild plant populations in their agronomic performance, such as yield, plant uniformity, stability, etc. Crop plants thus exclude wild plant populations, which occur naturally and which evolved without human intervention.

"Diploid plant" refers to a plant, vegetative plant part(s), or seed from which a diploid plant can be grown, having two sets of (homologous) chromosome, designated herein as 2n. Traditionally Corn salad is a diploid species with 2n = 16 chromosomes, i.e. with 8 chromosome pairs in vegetative cells. Haploid cells (male and female gametes) have n = 8 chromosomes. Examples of diploid Corn salad varieties are, for example, varieties "Vit" and "Valentin".

"Tetraploid plant" refers to a plant, vegetative plant part(s), or seed from which a tetraploid plant can be grown, having four sets of chromosomes, designated herein as 4n. A tetraploid Corn salad plant has 4n = 32 chromosomes, i.e. 2 × 8 (homologous) chromosome pairs in vegetative cells. Haploid cells (male and female gametes) have 2n = 16 chromosomes. Examples are A-1, B-1, B-2 and B-3 described herein.

"Autotetraploid" has chromosome sets derived from a single species, e.g. through genome duplication of a diploid plant.

An "induced tetraploid plant" or "induced tetraploid cell" is generated by human intervention which results in chromosome / genome doubling of a diploid cell, such as by *in vitro* culture (and spontaneous chromosome doubling in culture), chemical treatment to induce chromosome doubling, e.g. using colchicine (or other anti-mitotic agents), oryzalin (or other herbicides which induce chromosome doubling), nitrous oxide gas, trifluralin, pronamide, etc., and/or physical treatments (e.g. temperature, radiation, etc.).

"Planting" refers to seeding (direct sowing) or transplanting seedlings/plantlets into a field by machine or hand. Planting may also encompass seeding or transplanting seedlings/plantlets in soilless or hydroponic culture systems.

The verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one", e.g. "a plant" refers also to several plants, etc. Similarly, "a leaf' refers to a plurality of leaves.

As used herein, the term "plant" includes the whole plant or any parts or derivatives thereof, preferably having the same genetic makeup as the plant from which it is obtained, such as plant organs (e.g. harvested or non-harvested leaves, etc.), plant cells, plant protoplasts, plant cell tissue cultures from which whole plants can be regenerated, plant calli, plant cell clumps, plant transplants, seedlings, plant cells that are intact in plants, plant clones or micropropagations, or parts of plants (e.g. harvested tissues or organs), such as plant cuttings, embryos, pollen, ovules, fruits, flowers, leaves, seeds, clonally propagated plants, roots, stems, root tips, grafts and the like. Also any developmental stage is included, such as seedlings, cuttings prior or after rooting, mature plants or leaves, etc.

"Harvested plant material" refers herein to whole plants and/or plant parts (e.g. leaves detached from all or part of the roots) which have been collected for further processing, such as one or more of washing, rinsing, drying, mixing with other plant material and/or other edible material, packaging, etc. and/or which have already undergone one or more further processing steps.

"Harvested seeds" refers to seeds harvested from a line or variety, e.g. produced after self-fertilization or cross-fertilization and collected.

"Fresh-leaf products" refers to products comprising or consisting of fresh (raw; not cooked or steamed) leaves, leaf clusters, plantlets or leaf-parts or plantlet-parts. For example containers (bags, punnets, packets, flow packs, etc.) comprising Corn salad, e.g. salad mixes.

As used herein, the term "variety" or "cultivar" means a plant grouping within a single botanical taxon of the lowest known rank, which grouping, irrespective of whether the conditions for the grant of a breeder's right are fully met, can be defined by the expression of the characteristics resulting from a given genotype or combination of genotypes, distinguished from any other plant grouping by the expression of at least one of the said characteristics and considered as a unit with regard to its suitability for being propagated unchanged.

"Plant line" is for example a breeding line which can be used to develop one or more varieties.

"Average" refers herein to the arithmetic mean.

### DETAILED DESCRIPTION OF THE INVENTION

### Plants according to the invention

In one embodiment of the invention a plant of the species *Valerianella locusta* is provided wherein the plant is (auto)tetraploid and comprises the duplicated genome of the diploid plant from which it is derived. Thus, the vegetative cells of the plant (e.g. the leaves) contain double the amount of nuclear DNA, i.e. 4n = 32 chromosomes, as can be determined by flow cytometry or other methods (see Dolezel and Bartos, Annals of Botany 2005 95(1):99-110). The tetraploid plants are generated by doubling the chromosomes of cultivated diploid *V. locusta* breeding lines or varieties, i.e. not by tetraploidization of wild *V. locusta,* as cultivated material has good agronomic characteristics and have the characteristics set out in the appended claims.

By tetraploidization of cultivated *V. locusta* plants, plants are modified in a number of features (relative to the diploid from which they are derived by chromosome doubling). In one embodiment of the invention the tetraploid plant has thicker and/or firmer and/or heavier (more liquid and less soluble solids per unit leaf area) leaves than the diploid plant from which it is derived. Preferably mature leaves of the same age and grown under the same conditions are used in the analyses described below. It is understood that for statistical purposes several leaves from a number of plants per line or cultivar are used to account for plant-to-plant and leaf-to-leaf variation within a line or cultivar.

The tetraploid plant has an average leaf thickness (of individual leaves and/or of a plurality of leaves) which is at least about 105%, preferably at least about 110%, preferably at least about 115%, 120%, 125%, 130%, 135%, 140%, 150% (or more) of the average leaf thickness of the diploid plant from which it was derived through chromosome doubling. Average leaf thickness (or average thickness of a plurality of leaves) can be determined using various means. For examples a plurality of leaves (e.g. at least about 3, 4, 5, 6, 7, 8, 9, 10, 15 or more) can be layered on top of one another, between slides, and thickness can be measured using a calliper. The average thickness of the leaves is compared to that of the diploid control (the diploid from which the tetraploid was derived), measured in the same way. Alternatively, microscopic method can be used to measure leaf thickness and compare this to the diploid control. For example the thickness of transverse leaf sections may be measured microscopically.

The leaves of the tetraploids are in one embodiment also firmer (stronger) than those of the diploids from which they are derived. Mechanical leaf strength can, for example, be measured by measuring tensile strength at the location of maximal leaf blade width with a Texture Analyzer, e.g. TA-XT2 (Texture Technologies, Scarsdale, NY). Tensile breaking strength is measured as force in Newtons (N) mm⁻² of transverse leaf blade area. Alternatively, a penetrometer can be used to measure leaf strength. The average leaf strength of tetraploid leaves is significantly higher than of the diploid controls, preferably it is at least 105%, 110%, or more of the control (being set to 100%). Likewise manual touch can be used to compare leaf firmness, and tetraploid leaves feel firmer than the control diploids. Firmness can also be quantified manually, e.g. touching leaves between two fingers and scoring firmness e.g. on a scale of 1-10 (with 10 being very firm, with the diploid variety Valentin having a firmness score of 4. Tetraploids according to the invention have an average leaf firmness on a scale of 1-10 which it at least 1, 2, 3 or more points higher than of the diploid from which they were derived.

Leaves of the tetraploid plants according to the invention preferably are not only thicker than the diploids, but are also heavier per unit area and have less solids than the diploids. Average fresh weight of leaves per unit area (e.g. per plug) is significantly increased compared to diploid controls, being at 102%, 103%, 105%, 110%, 120%, 125%, 130% (or more) of the diploid control weight. Fresh weight can simply be measured by weighing freshly samples leaves or leaf parts of defined size. After drying, the solid content of the leaf / leaf part can be determined. Noticeably, tetraploid leaves / leaf parts have a significantly larger percentage of liquid and significantly lower percentage of solids per unit area compared to diploid controls, indicating that cells are significantly larger, with more cytosol and less cell-wall parts. This is confirmed by SEM microscopy. Thus, in one embodiment the percentage of solid matter per unit area is significantly lower in tetraploid leaves compared to the diploid controls, e.g. the percentage solid matter is 95% or less of that of the diploid control (e.g. 94%, 93%, 90%, 89%, 85%, or less, of the diploid control).

The upper (adaxial) epidermal cells and/or the palisade cells are on average significantly larger in the tetraploid leaves compared to the diploids from which they were derived. Optionally, also the lower epidermal cells are significantly larger in the tertaploids. "Larger" refers herein especially to cell height, from upper cell wall to lower cell wall. Thus, tetraploids have upper epidermal cells which have an average cell height of at least 105%, preferably at least 110%, 115%, 119%, or more, of the diploid from which the tertaploid was derived, and/or palisade cells which have an average cell height of at least 110%, 115%, 120%, or more, of the diploid from which the tertaploid was derived. Optionally, also the lower epiderminal cells have a cell height which is at least 110%, or more, of the diploid from which the tertaploid was derived. Cell size can, for example, be measured by Scanning Electron Microscopy (SEM).

The tetraploid plants and/or leaves are "compacter" than the diploid controls. "Compacter" means that average leaf length of the tetraploid is significantly shorter than that of the diploid from which it was derived and/or that the width : length ratio is significantly higher in the tetraploid compared to the diploid from which the tetraploid was derived. "Significantly shorter" refers to the leaf length being equal to or less than 95%, 90%, 85%, 80%, 75%, or less, of the length of the diploid control. Likewise, a significantly higher width : length ratio refers to a ratio which is at least 105%, 110%, preferably at least 115%, 120%, or more, of the ratio of the diploid control. Thus, the compacter tetraploid plant according to the invention has shorter leaves, and optionally wider leaves, than the diploid from which it was derived. The (average) leaf length and width can be measured manually (as for example described in the examples) or by using automatic area meters (using e.g. photocopied leaves or leaf segments, see Tsuda, 1999, Annals of Botany 84: 799-801). Leaf length and width is preferably measured by detaching leaves from the stem and measuring the length of the entire leaf, including the petiole (the part attaching the blade to the stem), i.e. from the petiole base to the leaf tip.

Thus, the tetraploid plant according to the invention does not have longer leaves than the diploid plant from which it was derived, and has significantly shorter leaves and optionally broader leaves.

The "diploid plant from which the tetraploid plant was derived" or "diploid control" refers herein to the diploid plant whose chromosomes were doubled by human intervention, resulting in the tetraploid plant according to the invention. Also provided herein are progeny of any of the tetraploid plants of the invention, which are stable tetraploids (i.e. retain a duplicated diploid genome), such as generations obtained by self fertilization (selfing) and/or tetraploids obtained by cross fertilizing (crossing) one tetraploid with another tetraploid (resulting in "hybrid tetraploids"). Hybrid tetraploids are, thus, tetraploids which contain chromosomes originating from different diploid lines or varieties (but these hybrid tetraploids are still autotetraploids, as only chromosomes of *V. locusta* are present).

Molecular techniques, such as AFLP fingerprinting (Muminovic et al. Plant Breeding, Volume 123 Issue 5, Pages 460 - 466), RFLP fingerprinting, SNP markers or whole genome sequencing, can be used to determine which diploid plant(s) has/have been doubled in order to generate the tetraploid(s) and/or hybrid tetraploids according to the invention. Also triploid Corn salad plants can be made, by crossing a tetraploid with a diploid.

Basically, any diploid breeding line or cultivar/variety can be used to generate a tetraploid plant according to the invention, such as, but not limited to, commercial varieties. Examples of commercial varieties which can be used are Hild varieties (Baron, Granon, Eurion, Medaillon, Rodion, Elan, Vit, Valentin, Verte de Cambrai Caombrain/Hilmar), Rijk Zwaan varieties (Cirilla RZ, Dione RZ, Pulsar RZ), ENZA varieties (Accent, Favor, Juwallon, Juwahit, Juvert), Clause varieties (Trophy, Gala) and others. The diploid plant can be a variety or breeding line of *V. locusta* selected from a rosette type, a long leafed type, a plant adapted for winter or for summer cultivation, or a variety adapted for all year long cultivation. Varieties adapted for winter- or summer- cultivation can be harvested in the European or North American winter- or summer months, respectively. Varieties for all year long cultivation can be harvested throughout the year.

Preferably a stable *V. locusta* breeding line or variety, having good agronomic and/or quality characteristics, is used for tetraploidisation. Thus, preferably the line or variety used has good quality and/or agronomic characteristics, such as disease and/or pest resistance (e.g. *Phoma valerianellae, Peronospora valerianellae, Acidovorax valerianellae, Erisyphe* sp., *Pseudomonas* sp.), not susceptible to spoon formation of the leaves, high yield, dark green color, uniformity, low prominence of the veins, etc. In particular tetraploids having dark green leaves are preferred, as dark color is attractive to consumers.

In one embodiment of the invention, the tetraploids have a darker green leaf color than the diploids from which they were derived. Tetraploids with darker green leaves can be made by using diploid lines or varieties having light green, medium green or dark green leaves for tetraploidization. The tetraploids according to the invention have, in one embodiment, a color score which is at least 0.5, preferably at least 1, 1.5, 2, or more, scores higher than of the control diploid, e.g. on a scale of 1 (light green) to 10 (dark green). Tetraploids according to the invention preferably have a leaf color score of at least about 6, more preferably at least about 7 or 8, most preferably at least about 9 or 10. The diploid variety Vit, for example, scores about 7.7 on a scale of 1-10.

After tetraploidization, the selected 4n plant may be used as such for seed production, i.e. the tetraploid is selfed (to produce an S1) and seeds are collected. To upscale seed production the S1 seeds may be planted, selfed and S2 seeds may be collected from the S1 plants. Likewise S2, S3, S4 or other generations derived from the selected tetraploid may be used for seed and/or crop production.

Alternatively, tetraploids may be selfed and/or crossed with other tetraploids one or more times. In a particular embodiment the plants are selfed one or more times, to produce stable lines. The plants may be used in normal Corn salad breeding programs and further improvements may be made through breeding and selection. Thus, plants may be evaluated for morphological and agronomic characteristics and tetraploids with good agronomic performance and/or quality traits may be made using traditional breeding methods. Preferably the distinguishing phenotypic characteristics of the tetraploids (leaf thickness and/or compactness and/or firmness and/or crispiness) are retained in the progeny.

The tetraploid plants according to the invention are in one embodiment particularly suitable for summer cultivation.

The tetraploid plants according to the invention are generated by human intervention, using any chromosome doubling technique, such as treatment of cells, tissue or plantlets with anti-mitotic agents (e.g. colchicines) or microtubule depolymerising herbicides, and regeneration of tetraploid cells and plants. Chromosome doubling techniques and plant regeneration techniques are well known in the art.

In a further embodiment not according to the invention present for illustration purposes only, a tetraploid plant derived from seeds deposited under accession number NCIMB 41651 and/or NCIMB 41652, or from progeny thereof, is provided. Plants grown from seeds having accession number NCIMB 41651 and NCIMB 41652 or derived thereform (e.g. progeny produced by selfing and/or crossing) are tetraploid lines disclosed hereby but not according to the invention and present for illustration purposes only, . These two lines, and progeny thereof obtained by selfing and/or by crossing one of these with another tetraploid (e.g. hybrids obtainable by crossing these lines with one another) are particularly suitable for summer cultivation. Seeds and harvested plants and/or plant parts of the plants derived from the deposited seeds are also an embodiment herein. that are not according to the invention and are present for illustration purposes only,

In one aspect a seed of a tetraploid corn salad plant is disclosed, of which a representative sample of seed was deposited under accession number NCIMB 41651 or NCIMB 41652. Also a corn salad plant or plant part produced by growing these seeds is disclosed. Further, a tissue culture produced from cells or protoplasts of these plants is disclosed, wherein the cells or protoplasts are produced from a plant part, e.g. selected from the group consisting of leaf, pollen, embryo, cotyledon, stem, root, meristematic cell, root tip, pistil, anther, flower, shoot or seed. Also a plant regenerated from the tissue culture is disclosed, wherein the plant has all of the physiological and morphological characteristics of NCIMB 41651 or NCIMB 41652.

Seeds from any of the tetraploid plants according to the invention can be treated (e.g. coated, primed, etc.) and/or packaged using any known techniques. Seed packages preferably only comprise a single inbred tetraploid line or variety, or only a single "hybrid tetraploid" line (see above).

### Products according to the invention

Fresh and/or steamed and/or (partially)cooked products comprising or consisting of tetraploid plants (or plantlets) and/or tetraploid plant parts (e.g. leaves) are also an embodiment of the invention. Thus, in one embodiment a plurality of harvested leaves and/or harvested plants and/or plant parts of a plant according to the invention is provided. These harvested plants/parts are in one embodiment packaged. Optionally, prior to packaging plant material is washed and/or rinsed one or more times to remove debris. The tetraploid material may be mixed with other ingredients, for example other vegetables, such as other Corn salad material, *Lactuca sativa* material (e.g. baby leaf or multileaf lettuce), Rucola (Rocket) leaves, etc. Also ready-to-eat salads and meals comprising or consisting of tetraploid plants/parts are provided. Such ready-to-eat salads and meals may be vegetarian or may comprise meat and/or seafood.

In another embodiment a container or package comprising a plurality of fresh, harvested leaves is provided. The containers or packages may be open or sealed, they may be cartons, bags, punnets, trays, flow pack, film, etc. Preferably harvested leaves and products comprising these are kept cool during post harvest processing and/or packing and/or the subsequent supply chain (up to an including e.g. the supermarket shelf), preferably below 20 °C, more preferably below 15 °C, more preferably below 10 °C, e.g. at about 0 to 7 or 8 °C, most preferably about 2 - 4 °C (see e.g. Geyer and Herppich, *supra).* Protective packaging can be used to control moisture and/or gas compositions. Preferably the relative humidity (RH) in the package is at least about 90% or 95%, more preferably 100%. Atmosphere may be controlled, for example, through EMAP packaging (Equilibrium Modified Atmosphere Packaging) or anaerobic EMAP packaging (AEMAP). Every product has its ideal temperature, O₂, CO₂ and RH level, which can be determined using known methods.

In a further embodiment, the plants according to the invention and the products comprising or consisting of fresh tetraploid plants and/or plant parts (e.g. leaves) have a longer shelf-life and/or crisper (crunchier) mouthfeel than those comprising or consisting of fresh diploid Corn salad plants and/or plant parts (e.g. leaves), especially compared to the diploids from which the tetraploids were derived. In general, the shelf-life of fresh-cut leafy vegetables is 5-7 days from packaging, but shelf life depends on a large number of factors, such as the plant line or variety and harvest and post-harvest handling and packaging conditions, as well as storage and transport conditions.

"Longer shelf-life" means that the product stays marketable (i.e. retains a certain minimal quality) for a significantly longer period of time (postharvest, under the same packaging and storage conditions) than the equivalent product made using the diploid from which the tetraploid was derived. A significantly longer period is at least 1 day, preferably at least 2, 3, 4, 5, 6, 7 days longer, or more.

As tetraploid plants have thicker leaves compared to the diploid controls, the longer shelf-life compared to the diploid controls may only be achieved in minimal processing chains, where careful handling is used to minimize tissue damage. The skilled person can establish harvesting and post-harvest handling chains which result in a longer shelf-life, for example by including more manual handling steps and reducing mechanical steps.

A product becomes unmarketable when quality indicators, such as color, tissue decay/rot and/or tissue firmness is not acceptable anymore to the consumer. The post-harvest quality can be compared, for example, by scoring post-harvest quality indicators (such as leaf color, rot and/or tissue firmness) at various time points after storage of harvested leaves or plantlets (e.g. stored for 2, 4, 6, 8 days at cool temperatures). See for example Nicola, Hoebrechts and Fontana, Acta Hort. 633, ISHS 2004, p509-515.

A "crispier" mouthfeel or "crunchier" mouthfeel or "firmer" mouthfeel compared to the diploid controls can be tested using a taste panel, whereby persons are asked to score mouthfeel on a defined scale, e.g. with 1 being not crispy/firm and 10 being the most crispy/firm. Thus, for example, leaves derived from plant A-1 (NCIMB 41651) are on average crispier than leaves of variety A. Likewise, leaves derived from plant B-3 (NCIMB 41652) are on average crispier than leaves of variety B. Thus, in general, leaves of tetraploids are crispier/crunchier than leaves of the diploid from which they were derived.

Also vegetative propagation of tetraploids, e.g. through cell or tissue cultures, is possible. Tissue cultures, such as callus and suspension cultures have, for example, been described by Schrall and Becker (Acta Horticulturae 1980, Vol. 96: 75-83). A vegetative propagation (protoplast-, cell- or tissue culture) of a tetraploid according to the invention is, therefore, also an embodiment herein, as well as plants regenerated therefrom, progeny or seeds thereof.

Further SCS (soilless culture systems) based on growing media have been described and can be applied to tetraploids, see Fontana and Nicola *(supra).*

In yet a further embodiment of the invention a method for producing tetraploid plants (or plant parts thereof) of the species *Valerianella locusta* is provided, comprising:
a) providing a diploid plant cell, tissue, organ, seed or plantlet of the species *Valerianella locusta,*
b) inducing chromosome doubling in said plant cell, tissue, organ, seed or plantlet,
c) producing a tetraploid plant from b).

The plants or vegetative plant parts of these derived from step c) are also tetraploid. It is understood that this method is suitable for providing plants (and plant parts) with the characteristics described earlier (thicker leaves, compacter plants, etc. compared to the original diploid used in step a). The plants obtained from step c) may be used to grow a Corn Salad crop in the field or greenhouse and for harvesting plants or plant parts thereof.

The diploid plant cell, tissue, organ, seed or plantlet of step a) may be of any diploid *V. locusta* line or variety, preferably a cultivated *V. locusta,* most preferably a line or variety having good agronomic characteristics.

The seeds may for example be germinated seeds. Chromosome doubling can be induced by various methods, e.g. by oryazlin treatment as described in the examples. In step c) the plant(s) is/are regenerated *in vitro* or allowed to grow (e.g. from germinated seeds or plantlets). Tetraploid plants can be selected using e.g. flow cytometry and/or through selection of plants having thick leaves (e.g. through manual selection by feeling the leaves). Diploids or chimeras can be discarded.

Also haploids or double haploids may be produced from the tetraploids according to the invention. The method for producing haploids or double haploids comprises:
a) providing a haploid plant cell or cell line of a tetraploid plant of the species *Valerianella locusta,*
b) optionally inducing chromosome doubling in said plant cell or cell line to produce a double haploid plant cell or cell line,
c) producing a haploid plant from the cell or cell line of a) or a double haploid plant from the cell or cell line of b), and, optionally,
d) further crossing and/or selfing with the plant of c) and/or with progeny thereof.

The haploid cell or cell line may for example be an anther culture of the tetraploid.

Plants, plant parts and seeds produced by the above methods are encompassed herein.

### DEPOSIT INFORMATION

The tetraploid line derived from diploid variety A was named A-1 and corresponds to line NUN 0008, of which 2500 seeds were deposited under the Budapest Treaty by Nunhems B.V. on September 4^{th}, 2009 at the NCIMB (Ferguson Building, Craibstone Estate, Bucksburn, Aberdeen, AB21 9YA, Scotland). These seeds were assigned deposit number NCIMB 41651.

The three tetraploid lines derived from diploid variety B were named B-1, B-2 and B-3. Line B-3 corresponds to line E09_T0981-17, of which 250 seeds were deposited by Nunhems B.V. under the Budapest Treaty on 4 September 2009 at the NCIMB (Ferguson Building, Craibstone Estate, Bucksburn, Aberdeen, AB21 9YA, Scotland). These seeds were assigned deposit number NCIMB 41652.

Access to the seeds is governed by the applicable articles and rules of the Budapest Treaty and the Applicant chooses the 'expert' solution of Rule 32 of the European Patent Convention (EPC 2000).

The following non-limiting examples describe the production of tetraploid *V. locusta* according to the invention.

### EXAMPLES

### Example 1 - Tetraploidization and Flow Cytometry

The commercial diploid (2n) *V. locusta* varieties A and B were treated with oryzalin to induce chromosome doubling. Variety A is obtainable commercially from Hild Samen GmbH, Kirchenweinbergstr. 115, D-71672 Marbach, Germany, under the name "Vit"). Variety B is obtainable commercially from Hild Samen under the name "Valentin".

Variety A is a veined, dark-green, oval-leafed "rosette" variety, suitable for autumn and winter cultivation under glass or plastic covers. Variety B is "rosette" type variety with dark green, strong, broad and round leaves having a short stem, suitable for summer cultivation.

The following protocol was used. Seeds of variety A and B were allowed to germinate at 21 degrees Celsius on seed germination paper. Once more than 50% of seeds were germinated, germinated seeds were transferred into a sieve. Sieves were submerged for 4-6 hours at room temperature in an oryzalin solution (5 mg per litre tapwater). Sieves were washed in tapwater three times for about 15 minutes. Germinated seeds were transferred into soil, covered with vermiculite and left to germinate further. Leaf samples were taken per plant and analyzed by Flow Cytometry to determine ploidy levels. Flow Cytrometry analysis was carried out by Plant Cytometry Services (Europalaan 74, 5481 JG Schijndel, The Netherlands).

One tetraploid line, designated A-1, derived from diploid variety A, was selected for further analysis. Three tetraploid lines, designated B-1, B-2, and B-3, derived from variety B, were selected for further analysis.

### Example 2 - Phenotypic analysis of tetraploids vs. diploids

### 2.1 Leaf characteristics

### 2.1.1 - Material and Methods

Seeds of variety A and B, as well as A-1, B-1, B-2 and B-3 were sown in a climate chamber. After 2 weeks of cold imbibition at 5 °C in the dark the seeds were allowed to germinate at 18 °C with 12 hours day/night under artificial illumination for two weeks. After 2 weeks of seed germination and seedling development plants were vernalized at 5 °C, 12 hours day/night under artificial illumination during 4 weeks. Beginning of July they were planted in the field under green netting for final development. Plants were assessed visually and manually for phenotypic characteristics.

At maturity (7-8 leaf pair stage), from 20 plants per variety/line fresh leaves were taken between 8:00 and 9:00 am. Leaves were carefully washed to remove sand and to keep leaves fresh. A paper towel was used to remove (surface) water.

Two leaf plugs of 1.2 cm diameter were removed from each of two leaves per variety/line and weighed to determine average fresh weight (leaf plug weights 1-10 in Table 1). Plugs were taken from one half of the leaf, avoiding the main vein.

The other half of the leaves was cut to remove the main vein (and the half from which leaf plugs had been taken). This half was used to determine average leaf thickness (Table 3) and percent solid matter (Table 2), i.e. the percentage of the fresh weight which is composed of dry matter (solids). To determine dry matter, the 10 leaf halves were placed into a drying oven for 3.5 hrs at 120 °C and weighed subsequently.

To determine average leaf thickness (Table 3) the 10 leaf halves were laid on top of each other and placed between two plastic slides. The thickness of the 10 leaves was measured twice with a (digital) calliper (measurement 1 and 2 in Table 3). All samples were measured in the same way by applying light pressure (until resistance was felt).

### 2.1.2 - Results

### Visual/manual assessment

Visual and manual assessment showed that the plants of the tetraploid A-1, B-1, B-2 and B-3 were compacter and had seemingly thicker leaves than the diploids from which they were derived (A and B). Leaf thickness and toughness were a phenotypic feature which could be used (by taking the leaf between thumb and finger) to distinguish the tetraploids from the diploids. One line, B-4 (derived from variety B) did not seem to have thicker leaves than B and plants were not compacter than B. It was, therefore, suspected that this plant line B-4 was in fact not a tetraploid but a diploid. This was later confirmed by flow cytometry and the plants were discarded from further analysis.

In order to check whether the initial visual and manual assessment could be verified and quantified, various measurements were done as described above.

### Fresh weight

Average leaf fresh weight of the tetraploids was significantly higher than in the diploids from which they were derived (Table 1; student's t-test, two-tailed, type two-sample equal variance). In plug measurements average fresh weight of tetraploid line A-1 was 125% of diploid line A, and average fresh weight of tetraploid lines B-1, B-2 and B-3 was 126%, 122% and 122% of that of diploid line B, respectively (Table 1).

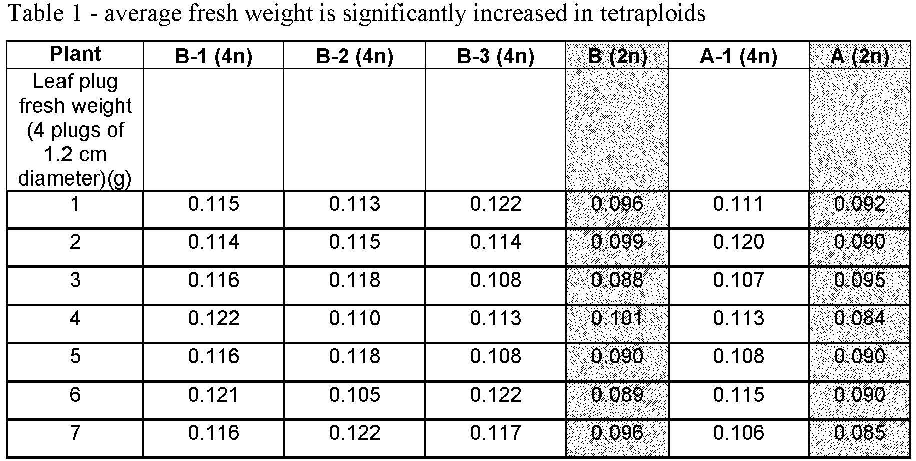

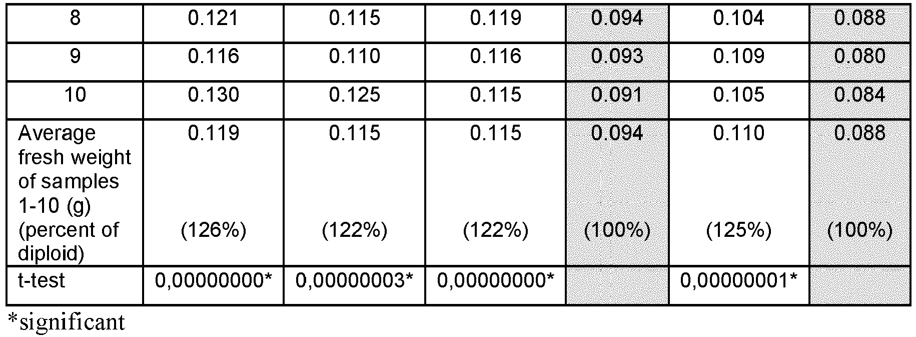

These data confirmed the finding that leaves of tetraploids are thicker and therefore significantly heavier per unit area than the diploid controls.

### Percentage dry matter

The (average) percentage dry matter was significantly lower in the tetraploids than in the diploids from which they originated (Table 2; students t-test, as above), indicating that more liquid was present per leaf area, probably through larger cells and relatively less cell-walls being present per unit area.

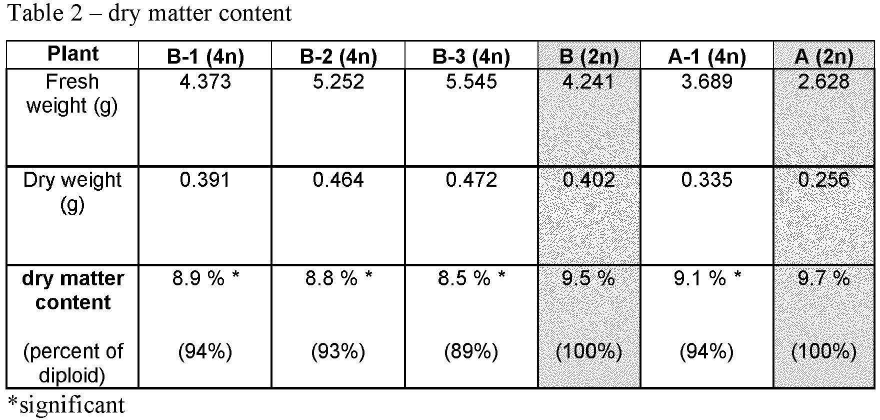

### Leaf thickness

Also the leaf thickness was significantly increased in the tetraploids compared to the diploids, see Table 3. As measurements could not be carried out on single leaves, measurements were made for 10 leaves per line as described above.

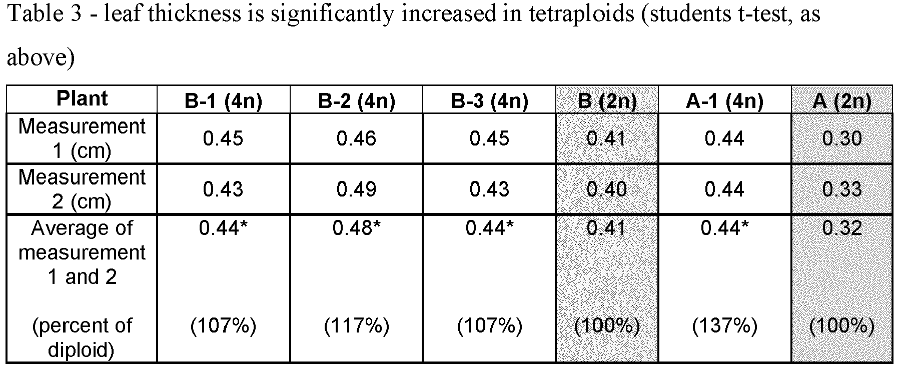

### 2.1.3 - Conclusions

Both fresh weight and leaf thickness measurements confirmed that tetraploidization leads to significantly thicker and heavier leaf tissue per unit area. As dry matter was found to be significantly lower, this increase is due to larger cells being present in the tetraploids, rather than more cells being present.

### Example 3 - Microscopic analysis

Transverse leaf sections were cut and photographs were taken using a binocular microscope. On the photographs, leaf thickness was measured at a distance of 500 and 1000 µm from the leaf tip. Leaves of tetraploids were significantly thicker than leaves of the diploids from which they were derived, as shown below:

**Table 4**

| | Leaf thickness (µm) measured at 500 µm from tip | Leaf thickness (µm) measured at 1000 µm from tip |
|---|---|---|
| A (2n) | 250 | 250 |
| A-1 (4n) (NUN0008; NCIMB 41651) | 320 | 390 |
| B (2n) | 270 | 290 |
| B-3 (4n) (E09_T0981-17; NCIMB 41652) | 320 | 390 |

### Example 4 - Compactness of tetraploids

To determine compactness, 10 mature leaves were removed from a minimum of 5 plants per line. Leaves were placed on millimetre paper and measured. The results are shown below in Table 5 and Table 6.

**Table 5**

| | A-1 (4n) | | | A (2n) | | |
|---|---|---|---|---|---|---|
| | Leaf width (cm) | Leaf length (cm) | Ratio width : length | Leaf width (cm) | Leaf length (cm) | Ratio width : length |
| | 3,4 | 6,4 | 0,53 | 3,3 | 6,5 | 0,51 |
| | 3,3 | 6,6 | 0,50 | 3,1 | 6,5 | 0,48 |
| | 3,5 | 6,2 | 0,56 | 2,9 | 6,5 | 0,45 |
| | 3,2 | 6,2 | 0,52 | 2,8 | 7,2 | 0,39 |
| | 3,3 | 5,8 | 0,57 | 2,8 | 5,8 | 0,48 |
| | 3,3 | 6,0 | 0,55 | 3,1 | 6,8 | 0,46 |
| | 3,2 | 5,8 | 0,55 | 2,9 | 6,5 | 0,45 |
| | 3,3 | 6,2 | 0,53 | 2,7 | 5,8 | 0,47 |
| | 3,5 | 6,3 | 0,56 | 3,0 | 6,9 | 0,43 |
| | 3,3 | 6,2 | 0,53 | 2,9 | 6,8 | 0,43 |
| Average (% of A) | 3,3 (110%) | 6,2 (95%) | 0,54 (120%) | 3,0 (100%) | 6,5 (100%) | 0,45 (100%) |
| t-test | 0,000* | 0,043* | 0,0000* | | | |

**Table 6**

| | B-3 (4n) | | | B (2n) | | |
|---|---|---|---|---|---|---|
| | Leaf width (cm) | Leaf length (cm) | Ratio width : length | Leaf width (cm) | Leaf length (cm) | Ratio width : length |
| | 3,8 | 5,9 | 0,64 | 4,0 | 7,0 | 0,57 |
| | 3,6 | 5,0 | 0,72 | 3,6 | 6,4 | 0,56 |
| | 3,3 | 5,2 | 0,63 | 3,6 | 6,3 | 0,57 |
| | 3,7 | 5,5 | 0,67 | 3,5 | 6,3 | 0,56 |
| | 3,4 | 5,2 | 0,65 | 3,6 | 7,0 | 0,51 |
| | 3,5 | 5,8 | 0,60 | 3,7 | 6,8 | 0,54 |
| | 3,5 | 5,0 | 0,70 | 3,8 | 6,8 | 0,56 |
| | 4,1 | 5,2 | 0,79 | 3,8 | 6,7 | 0,57 |
| | 3,8 | 5,0 | 0,76 | 4,0 | 7,0 | 0,57 |
| | 4,1 | 5,0 | 0,82 | 3,7 | 6,2 | 0,60 |
| Average (% of B) | 3,7 (100%) | 5,3 (79%) | 0,70 (125%) | 3,7 (100%) | 6,7 (100%) | 0,56 (100%) |
| t-test | 0,63 | 0,0000* | 0,0001* | | | |

| | | | | | | |
|---|---|---|---|---|---|---|
| ^{∗}significant | | | | | | |

As can be seen, tetraploids have significantly shorter leaves and a significantly higher width : length ratio than the diploid from which they are derived (significance was determined using the t-test, two-tailed, type two-sample unequal variance).

Tetraploid A-1 also has a significantly wider leaf blade than A. Tetraploid B-3 has significantly shorter leaves compared to B, however without the leaf width being significantly different.

### Example 5 - Scanning Electron Microscopy (SEM)

### 5.1 Material and methods

In order to determine whether the increase in leaf thickness through tetraploidization is indeed due to larger cells (and not more cells), SEM pictures were taken and cell sizes were measured (i.e. cell height, the distance from upper cell wall to lower cell wall per cell; in pixels).

From mature leaves of equal size a piece of 3x3mm was cut out next to the main vein. The piece was put into the slot of a slotted stub and fastened with carbon-rich conductive glue, with the top 1 mm sticking out. The stub was flash frozen in liquid nitrogen. After transfer to the pre-chamber of a JEOL 6330 cryo FESEM the top of the leaf piece was fractured at -120°C. Subsequently a sublimated step at -90°C was done and a layer of gold-palladium was applied to the sample. Inside the microscope the leaf surface was studied and, where the fracture was perpendicular to the leaf surface, photographs were taken.

With ImageJ software the photos were examined and measurements of cell height were done. The height of cells of the upper- and lower epidermis and of the palisade parenchyma layer directly below the upper epidermis were measured. All measurements represent cell dimensions perpendicular to the leaf surface. Using the above mentioned technique a little shrinkage occurs, but because samples are prepared simultaneously differences are representative for differences between untreated samples. 212 pixels represented 100 µm, which is used to convert pixels into actual values.

### 5.2 Results

Results are shown in Table 7 (diploid A and tetraploid A-1) and Table 8 (diploid B and tetraploid B-3).

**Table 7 - Cell height of leaf tissue of diploid A and tetraploid A-1**

| Plant | Upper epidermis Cells (pixels) | Lower epidermis Cells (pixels) | Palisade parenchyma cells (long side) (pixels) |
|---|---|---|---|
| A | 116,92 | 81,10 | 95,60 |
| | 93,72 | 78,30 | 100,58 |
| | 110,34 | 95,79 | 90,32 |
| | 70,68 | 83,74 | 71,62 |
| | 86,21 | 90,32 | 85,80 |
| | 66,24 | 75,81 | 71,02 |
| | 57,40 | 92,04 | 69,79 |
| | 68,12 | 85,80 | 88,41 |
| | 72,75 | 73,44 | 73,37 |
| | 65,60 | 66,20 | 83,23 |
| | 89,37 | 75,81 | 86,35 |
| | 79,74 | 90,23 | 83,53 |
| | 76,42 | 77,16 | |
| | 77,03 | 80,54 | |
| | 64,73 | 64,62 | |
| | | 83,06 | |
| Average pixels | 79,68 | 80,87 | 83,30 |
| Average µm | 37,59 (100%) | 38,15 (100%) | 39,29 (100%) |
| | | | |

| Plant | Upper epidermis Cells (pixels) | Lower epidermis Cells (pixels) | Palisade parenchyma cells (long side) (pixels) |
|---|---|---|---|
| A-1 | 106,77 | 109,41 | 118,25 |
| | 73,95 | 90,68 | 102,21 |
| | 70,07 | 111,66 | 110,09 |
| | 103,09 | 108,13 | 120,19 |
| | 99,39 | 107,36 | 116,95 |
| | 116,01 | 115,20 | 102,53 |
| | 89,44 | 100,88 | 111,53 |
| | 93,49 | 103,73 | |
| | 80,40 | | |
| | 109,99 | | |
| | 107,26 | | |
| Average pixels | 95,44 | 105,88 | 111,68 |
| Average µm (% of A) | 45,02 (119%) | 49,94 (130%) | 52,68 (134%) |
| t-test | 0,023* | 0,000* | 0,000* |

| | | | |
|---|---|---|---|
| 212 pixels = 100 µm; ^{∗}significant | | | |

As can be seen, the cells of the lower and upper epidermis and the palisade tissue are on average significantly larger in the tetraploid, compared to the diploid from which the tetraploid was derived. Upper epidermis cells in the diploid ranged from 57 to 116 pixels, while in the tetraploid they ranged from 70 to 116 pixels. In the lower epidermis, the diploid cells had a height of 64 to 95 pixels, and the tetraploid from 90 to 115 pixels. The biggest difference was seen in the size of palisade cells (longitudinal), with 69 to 100 pixels in the diploid A compared to 102 to 120 pixels in the tetraploid A-1. The cells of A-1 are thus 119%, 130% and 134% the size (height) of A, for upper epidermis, lower epidermis and palisade cells, respectively. Palisade cells are the site of the leaf tissue with the largest number of chloroplasts per cell and where the main photosynthesis occurs. As tetraploids also appear to be darker green in colour compared to the diploids from which they are derived, and the palisade cells are larger, it appears that the teraploids have a larger number of chloroplasts than the diploids. Dark green colour is a desired quality trait for consumers.

**Table 8 - Cell height of leaf tissue of diploid B and tetraploid B-3**

| Plant | Upper epidermis Cells (pixels) | Lower epidermis Cells (pixels) | Palisade parenchyma cells (long side) (pixels) |
|---|---|---|---|
| B | 112,21 | 83,35 | 96,01 |
| | 92,24 | 76,11 | 98,68 |
| | 96,45 | 91,00 | 101,37 |
| | 88,70 | 75,64 | 85,37 |
| | 78,71 | 85,34 | 87,72 |
| | 102,53 | 85,52 | 88,49 |
| | 76,01 | | |
| | 86,92 | | |
| | 91,14 | | |
| Average pixels | 91,66 | 82,83 | 92,94 |
| Average µm | 43,23 (100%) | 39,07 (100%) | 43,84 (100%) |
| | | | |

| Plant | Upper epidermis Cells (pixels) | Lower epidermis Cells (pixels) | Palisade parenchyma cells (long side) (pixels) |
|---|---|---|---|
| B-3 | 129,35 | 81,34 | 109,71 |
| | 110,05 | 98,95 | 115,08 |
| | 126,05 | 78,85 | 111,63 |
| | 114,15 | 87,74 | 117,29 |
| | 109,44 | 101,54 | 104,21 |
| | 88,09 | 84,85 | 116,15 |
| | 91,69 | 97,15 | |
| | | 86,92 | |
| | | 92,04 | |
| Average pixels | 109,83 | 89,93 | 112,35 |
| Average µm (% of B) | 51,81 (119%) | 42,42 (108%) | 52,99 (120%) |
| t-test | 0,017* | 0,125 | 0,000* |

| | | | |
|---|---|---|---|
| 212 pixels = 100 µm;^{∗} significant | | | |

Table 8 gave a similar picture for the diploid B and the tetraploid B-3, except that no significant difference in average cell sizes of the lower epidermis were found. However, as in Table 7, both the upper epidermis cells and the palisade cells were significantly larger in the tetraploid compared to the diploid from which they are derived (average upper epidermis and palisade cells of B-3 were 119% and 120% of that of B).

Thus, it can be concluded that tetraploidization leads to significantly larger cells (on average) of the upper epidermis and the palisade tissue, and in certain cases also of the lower epidermis. It is thus this increase in cell size in the adaxial cell layers (upper epidermis and palisade cells) that makes the leaves thicker and firmer. The increase in cell size is likely also responsible for a crunchier/crispier mouthfeel when consumed and likely also gives the leaves a darker green colour.

## Claims

1. A plant of the species *Valerianella locusta,* wherein said plant is a tetraploid derived from a cultivated diploid *Valerianella locusta* breeding line or variety and wherein the average leaf length of the tetraploid is equal to or less than 95% of the diploid plant from which it was derived.

2. The plant according to claim 1, wherein the leaves have a significantly higher width : length ratio than the leaves of the diploid plant from which the tetraploid was derived.

3. The plant according to claim 2, wherein said leaves have an average width : length ratio which is at least 105%, preferably at least 110% of the width: length ratio of the diploid from which it was derived.

4. The plant according to any one of claims 1 to 3, wherein said plant has significantly thicker leaves than the diploid plant from which it is derived.

5. The plant according to claim 4, wherein said plant has an average leaf thickness which is at least 105%, preferably at least 110%, of the average leaf thickness of the diploid plant from which it was derived.

6. The plant according to any of claims 1 to 5, wherein said plant was generated by human intervention, preferably by induced chromosome doubling.

7. A plant according to any one of the preceding claims, wherein the diploid plant from which said tetraploid is derived is a variety or line of *V. locusta* selected from: a rosette type, a long leafed type, a variety or line adapted for winter cultivation, a variety or line adapted for summer cultivation, a variety for all year long cultivation.

8. A tetraploid plant according to any one of the preceding claims, obtained by crossing a tetraploid plant deposited under accession number NCIMB41651 or NCIMB41652, or progeny thereof, with another tetraploid plant.

9. A plurality of harvested leaves and/or harvested plants and/or plant parts of a plant according to any of claims 1 to 8.

10. The harvested leaves or harvested plants or plant parts of claim 9, wherein said leaves and/or plants and/or plant parts have a longer shelf-life and/or crisper mouthfeel than those derived from the diploid plant from which the tetraploid was derived.

11. Seeds from which a plant according to any one of claims 1 to 8 can be grown.

12. A cell or tissue culture of a plant according to any one of claims 1 to 8.

13. A method for producing tetraploid plants of the species *Valerianella locusta,* comprising:
a. providing a cultivated diploid plant cell, tissue, organ, seed or plantlet of a breeding line or variety of the species *Valerianella locusta,*
b. inducing chromosome doubling in said plant cell, tissue, organ, seed or plantlet,
c. producing a tetraploid plant from b).

14. The method according to claim 13, further comprising growing the tetraploid plants obtained from c) and harvesting the plants and/or plant parts.

## Patentansprüche

1. Pflanze der Spezies *Valerianella locusta,* wobei es sich bei der Pflanze um eine Tetraploide handelt, die von einer kultivierten diploiden *Valerianella locusta-*Züchtungslinie oder -sorte abgeleitet ist und wobei die mittlere Blattlänge der Tetraploiden gleich oder kleiner 95% der diploiden Pflanze, von der sie abgeleitet war, ist.

2. Pflanze nach Anspruch 1, wobei die Blätter ein signifikant höheres Breite:Länge-Verhältnis als die Blätter der diploiden Pflanze, von der die Tetraploide abgeleitet war, aufweisen.

3. Pflanze nach Anspruch 2, wobei die Blätter ein mittleres Breite:Länge-Verhältnis aufweisen, das wenigstens 105%, bevorzugt wenigstens 110% des Breite:Länge-Verhältnisses der Diploiden, von der sie abgeleitet war, beträgt.

4. Pflanze nach einem der Ansprüche 1 bis 3, wobei die Pflanze signifikant dickere Blätter als die diploide Pflanze, von der sie abgeleitet ist, aufweist.

5. Pflanze nach Anspruch 4, wobei die Pflanze eine mittlere Blattdicke aufweist, die wenigstens 105%, bevorzugt wenigstens 110%, der mittleren Blattdicke der diploiden Pflanze, von der sie abgeleitet war, beträgt.

6. Pflanze nach einem der Ansprüche 1 bis 5, wobei die Pflanze durch Eingriff des Menschen erzeugt wurde, bevorzugt durch induzierte Chromosomenverdopplung.

7. Pflanze nach einem der vorhergehenden Ansprüche, wobei es sich bei der diploiden Pflanze, von der die Tetraploide abgeleitet ist, um eine Sorte oder Linie von *V. locusta* handelt, die ausgewählt ist aus: einem Rosettentyp, einem langblättrigen Typ, einer an eine Winterkultivierung angepassten Sorte bzw. Linie, einer an eine Sommerkultivierung angepassten Sorte bzw. Linie, einer Sorte zur ganzjährigen Kultivierung.

8. Tetraploide Pflanze nach einem der vorhergehenden Ansprüche, erhalten durch Kreuzen einer unter Zugangsnummer NCIMB41651 oder NCIMB41652 hinterlegten tetraploiden Pflanze oder Nachkommen davon mit einer weiteren tetraploiden Pflanze.

9. Mehrere geerntete Blätter und/oder geerntete Pflanzen und/oder Pflanzenteile einer Pflanze nach einem der Ansprüche 1 bis 8.

10. Geerntete Blätter oder geerntete Pflanzen oder Pflanzenteile gemäß Anspruch 9, wobei die Blätter und/oder Pflanzen und/oder Pflanzenteile eine längere Haltbarkeit und/oder knackigeres Gefühl im Mund als solche, die von der diploiden Pflanze stammen, von der die Tetraploide abgeleitet war, aufweisen.

11. Samen, aus denen eine Pflanze nach einem der Ansprüche 1 bis 8 gezogen werden kann.

12. Zell- oder Gewebekultur einer Pflanze nach einem der Ansprüche 1 bis 8.

13. Verfahren zur Herstellung tetraploider Pflanzen der Spezies *Valerianella locusta,* umfassend:
a. Bereitstellen einer/eines kultivierten diploiden Pflanzenzelle, -gewebes, -organs, -samens oder Pflänzchens einer Züchtungslinie oder -sorte der Spezies *Valerianella locusta,*
b. Induzieren von Chromosomenverdopplung in der/dem Pflanzenzelle, -gewebe, -organ, -samen oder Pflänzchen,
c. Herstellen einer tetraploiden Pflanze aus b).

14. Verfahren nach Anspruch 13, ferner umfassend Kultivieren der aus c) erhaltenen tetraploiden Pflanzen und Ernten der Pflanzen und/oder Pflanzenteile.

## Revendications

1. Végétal de l'espèce *Valerianella locusta,* ledit végétal étant un tétraploïde issu d'une lignée généalogique ou d'une variété de *Valerianella locusta* diploïde cultivée et la longueur moyenne de feuille du tétraploïde étant égale ou inférieure à 95 % du végétal diploïde duquel il a été issu.

2. Végétal selon la revendication 1, les feuilles possédant un rapport largeur : longueur significativement plus grand que les feuilles du végétal diploïde duquel le tétraploïde a été issu.

3. Végétal selon la revendication 2, lesdites feuilles possédant un rapport moyen largeur : longueur qui est au moins 105 %, préférablement au moins 110 % du rapport largeur : longueur du diploïde duquel il a été issu.

4. Végétal selon l'une quelconque des revendications 1 à 3, ledit végétal ayant des feuilles significativement plus épaisses que le végétal diploïde duquel il est issu.

5. Végétal selon la revendication 4, ledit végétal possédant une épaisseur moyenne de feuille qui est au moins 105 %, préférablement au moins 110 %, de l'épaisseur moyenne de feuille du végétal diploïde duquel il a été issu.

6. Végétal selon l'une quelconque des revendications 1 à 5, ledit végétal ayant été généré par intervention humaine, préférablement par doublement chromosomique induit.

7. Végétal selon l'une quelconque des revendications précédentes, le végétal diploïde duquel ledit tétraploïde est issu étant une variété ou lignée de *V. locusta* choisie parmi : un type en rosette, un type à longues feuilles, une variété ou lignée adaptée à une culture hivernale, une variété ou lignée adaptée à une culture estivale, une variété pour une culture tout au long de l'année.

8. Végétal tétraploïde selon l'une quelconque des revendications précédentes, obtenu par croisement d'un végétal tétraploïde déposé sous le numéro d'accès NCIMB41651 ou NCIMB41652, ou d'une progéniture correspondante, avec un autre végétal tétraploïde.

9. Pluralité de feuilles récoltées et/ou de végétaux récoltés et/ou de parties de végétaux d'un végétal selon l'une quelconque des revendications 1 à 8.

10. Feuilles récoltées ou végétaux récoltés ou parties de végétaux selon la revendication 9, lesdites feuilles et/ou lesdits végétaux et/ou lesdites parties de végétaux possédant une durée de stockage plus longue et/ou une impression en bouche plus craquante que ceux/celles issu(e)s du végétal diploïde duquel le tétraploïde a été issu.

11. Semences à partir desquelles on peut faire pousser un végétal selon l'une quelconque des revendications 1 à 8.

12. Culture cellulaire ou de tissu d'un végétal selon l'une quelconque des revendications 1 à 8.

13. Procédé pour la production de végétaux tétraploïdes de l'espèce *Valerianella locusta,* comprenant :
a. la mise à disposition d'une cellule, d'un tissu, d'un organe, d'une semence ou d'une plantule de végétal diploïde cultivé d'une lignée généalogique ou d'une variété de l'espèce *Valerianella locusta,*
b. l'induction d'un doublement chromosomique dans ladite cellule, ledit tissu, ledit organe, ladite semence ou ladite plantule de végétal,
c. la production d'un végétal tétraploïde à partir de b) .

14. Procédé selon la revendication 13, comprenant en outre le fait de faire pousser les végétaux tétraploïdes obtenus de c) et la récolte des végétaux et/ou des parties de végétaux.
